# EUROPÄISCHE PATENTSCHRIFT

(11) **EP 1 257 670 B1**
(45) Veröffentlichungstag und Bekanntmachung des Hinweises auf die Patenterteilung: **20.02.2008**
(21) Anmeldenummer: 01915051.5
(22) Anmeldetag: 23.02.2001
(51) Int. Cl.: C12Q 1/68

(54) **VERFAHREN ZUR DETEKTION VON CYTOSIN-METHYLIERUNG IN DNA PROBEN**
METHOD FOR DETECTING CYTOSINE METHYLATION IN DNA SAMPLES
PROCEDE POUR DETECTER LA METHYLATION DE CYTOSINE DANS DES ECHANTILLONS D'ADN

(30) Priorität: 25.02.2000 DE 10010282
(43) Veröffentlichungstag der Anmeldung: 20.11.2002
(73) Patentinhaber: Epigenomics AG, 10178 Berlin (DE)
(72) Erfinder: OLEK, Alexander, 10115 Berlin (DE); BERLIN, Kurt, 14532 Stahnsdorf (DE)
(74) Vertreter: Schubert, Klemens
(86) Internationale Anmeldenummer: PCT/DE2001/000747
(87) Internationale Veröffentlichungsnummer: WO 2001/062960

(56) Entgegenhaltungen:
- WO-A-95/00669
- WO-A-98/44151
- WO-A-98/56952
- WO-A-99/28498
- WO-A-99/55905
- US-A- 5 605 798
- GRIGG G AND CLARK S: "Sequencing 5-methylcytosine residues in genomic DNA" BIOESSAYS, CAMBRIDGE, GB, Bd. 16, Nr. 6, Juni 1994 (1994-06), Seiten 431-436, XP002106411 ISSN: 0265-9247
- REIN ET AL: "Identifying 5-methylcytosine and related modifications in DNA genomes" NUCLEIC ACIDS RESEARCH, OXFORD UNIVERSITY PRESS, SURREY, GB, Bd. 26, Nr. 10, 1998, Seiten 2255-2264, XP002143106 ISSN: 0305-1048
- PAUL C L ET AL: "Cytosine methylation: quantitation by automated genomic sequencing and GENESCAN analysis" BIOTECHNIQUES, EATON PUBLISHING, NATICK, US, Bd. 21, Nr. 1, Juli 1996 (1996-07), Seiten 126-133, XP002143107 ISSN: 0736-6205
- NIEMEYER C M ET AL: "DNA MICROARRAYS**" ANGEWANDTE CHEMIE, VCH VERLAGSGESELLSCHAFT, WEINHEIM, DE, Bd. 38, Nr. 19, 1999, Seiten 3039-3043, XP000961724 ISSN: 0044-8249
- GASSEN ET AL.: "PCR: Grundlage und Anwendungen der Polymerase-Kettenreaktion" 1994, GUSTAV FISCHER VERLAG , STUTTGART * Seite 73 - Seite 75 *
- KWOK ET AL.: "Effects of primer-template mismatches on the polymerase chain reaction:Human immunodeficiency virus type 1 model studies" NUCLEIC ACIDS RESEARCH, Bd. 18, Nr. 4, 25. Februar 1990 (1990-02-25), Seiten 999-1005, XP002220845
- BOLDT ET AL.: "One-tube method for complete HPA-1 genotyping by PCR using sequence-specific primers" BRITISH JOURNAL OF HEMATOLOGY, Bd. 99, 1997, Seiten 968-973, XP001096153

### SEQUENZPROTOKOLL

<110> Epigenomics AG
<120> Verfahren zur Detektion von Cytosin-Methylierung in DNA-Proben
<130> E01-1179-WO
<140>
   <141>
<160> 6
<170> Patent In Ver. 2.1
<210> 1
   <211> 31
   <212> DNA
   <213> Künstliche Sequenz
<220>
   <223> Beschreibung der künstlichen Sequenz:Oligonukleotid
<400> 1
   attatgttgg agtagtagag tttaaatggt t 31
<210> 2
   <211> 32
   <212> DNA
   <213> Künstliche Sequenz
<220>
   <223> Beschreibung der künstlichen Sequenz:Oligonukleotid
<400> 2
   acttaacact tactatttaa atcacaaccc at 32
<210> 3
   <211> 21
   <212> DNA
   <213> Künstliche Sequenz
<220>
   <223> Beschreibung der künstlichen Sequenz:Oligonukleotid
<400> 3
   gttggatgtt gttgagaaac g 21
<210> 4
   <211> 21
   <212> DNA
   <213> Künstliche Sequenz
<220>
   <223> Beschreibung der künstlichen Sequenz:Oligonukleotid
<400> 4
   gttggatgtt gttgagaaat g 21
<210> 5
   <211> 18
   <212> DNA
   <213> Künstliche Sequenz
<220>
   <223> Beschreibung der künstlichen Sequenz:Oligonukleotid
<400> 5
   aaaaactaca aaaactct 18
<210> 6
   <211> 18
   <212> DNA
   <213> Künstliche Sequenz
<220>
   <223> Beschreibung der künstlichen Sequenz:Oligonukleotid
<400> 6
   aaaaactacg aaaactct 18

## Beschreibung

Die Erfindung betrifft ein Verfahren zum Nachweis von 5-Methylcytosin in genomischen DNA-Proben.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar.
Die vorliegende Erfindung beschreibt ein Verfahren zur Detektion des Methylierungszustandes genomischer DNA Proben. Das Verfahren kann gleichzeitig auch zum Nachweis von Punktmutationen und Single Nucleotide Polymorphisms (SNPs) genutzt werden.

5-Methylcytosin ist die häufigste kovalent modifizierte Base in der DNA eukaryotischer Zellen. Sie spielt beispielsweise eine Rolle in der Regulation der Transkription, beim genetischen Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Eine relativ neue und die mittlererweile am häufigsten angewandte Methode zur Untersuchung von DNA auf 5-Methylcytosin beruht auf der spezifischen Reaktion von Bisulfit mit Cytosin, das nach anschließender alkalischer Hydrolyse in Uracil umgewandelt wird, welches in seinem Basenpaarungsverhalten dem Thymidin entspricht. 5-Methylcytosin wird dagegen unter diesen Bedingungen nicht modifiziert. Damit wird die ursprüngliche DNA so umgewandelt, dass Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt wird. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulfit reagiert nur an einzelsträngiger DNA) verhindert und alle Fällungs- und Reinigungsschritte durch schnelle Dialyse ersetzt (Olek, A. et al., Nucl. Acids. Res. 1996, 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausenden von möglichen Methylierungsanalysen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannten Möglichkeiten, 5-Methylcytosine nachzuweisen, kann aus dem folgenden Übersichtsartikel entnommen werden: Rein, T., DePamphilis, M. L., Zorbas, H., Nucleic Acids Res. 1998, 26, 2255.

Die Bisulfit-Technik wird bisher bis auf wenige Ausnahmen (z. B. Zechnigk, M. et al., Eur. J. Hum. Gen. 1997, 5, 94-98) nur in der Forschung angewendet. Immer aber werden kurze, spezifische Stücke eines bekannten Gens nach einer Bisulfit-Behandlung amplifziert und entweder komplett sequenziert (Olek, A. und Walter, J., Nat. Genet. 1997, 17, 275-276) oder einzelne Cytosin-Positionen durch eine "Primer-Extension-Reaktion" (Gonzalgo, M. L. und Jones, P. A., Nucl. Acids Res. 1997, 25, 2529-2531, WO 9500669) oder einen Enzymschnitt (Xiong, Z. und Laird, P. W., Nucl. Acids. Res. 1997, 25, 2532-2534) nachgewiesen. Zudem ist auch der Nachweis durch Hybridisierung beschrieben worden (Olek et al., WO 9928498).

Weitere Publikationen, die sich mit der Anwendung der Bisulfit-Technik zum Methylierungsnachweis bei einzelnen Genen befassen, sind: Xiong, Z. und Laird, P. W. (1997), Nucl. Acids Res. 25, 2532; Gonzalgo, M. L. und Jones, P. A. (1997), Nucl. Acids Res. 25, 2529; Grigg, S. und Clark, S. (1994), Bioassays 16, 431; Zeschnik, M. et al. (1997), Human Molecular Genetics 6, 387; Teil, R. et al. (1994), Nucl. Acids Res. 22, 695; Martin, V. et al. (1995), Gene 157, 261; WO 9746705 und WO 9515373.

Eine Übersicht über den Stand der Technik in der Oligomer Array Herstellung läßt sich aus einer im Januar 1999 erschienen Sonderausgabe von Nature Genetics (Nature Genetics Supplement, Volume 21, January 1999) und der dort zitierten Literatur entnehmen.

Es existieren verschiedene Verfahren um DNA zu immobilisieren. Das bekannteste Verfahren ist die Festbindung einer DNA, welche mit Biotin funktionalisiert ist, an eine Streptavidin-beschichtete Oberfläche (Uhlen, M. et al. 1988, Nucleic Acids Res. 16, 3025-3038). Die Bindungsstärke dieses Systems entspricht der einer kovalenten chemischen Bindung ohne eine zu sein. Um eine Ziel-DNA kovalent an eine chemisch vorbereitete Oberfläche binden zu können, bedarf es einer entsprechenden Funktionalität der Ziel-DNA. DNA selbst besitzt keine Funktionalisierung, die geeignet ist. Es gibt verschiedene Varianten, in eine Ziel-DNA eine geeignete Funktionalisierung einzuführen: Zwei leicht zu handhabende Funktionalisierungen sind primäre, aliphatische Amine und Thiole. Solche Amine werden quantitativ mit N-Hydroxysuccinimidestern umgesetzt, und Thiole reagieren unter geeigneten Bedingungen quantitativ mit Alkyliodiden. Eine Schwierigkeit besteht im Einführen einer solchen Funktionalisierung in eine DNA. Die einfachste Variante ist die Einführung durch einen Primer einer PCR. Gezeigte Varianten benutzen 5'-modifizierte Primer (NH₂ und SH) und einen bifunktionalen Linker.

Ein wesentlicher Bestandteil der Immobilisierung auf einer Oberfläche ist ihre Beschaffenheit. Bis jetzt beschriebene Systeme sind hauptsächlich aus Silizium oder Metall. Eine weitere Methode zur Bindung einer Ziel-DNA basiert darauf, eine kurze Erkennungssequenz (z. B. 20 Basen) in der Ziel-DNA zur Hybridisierung an ein oberflächenimmobilisiertes Oligonukleotid zu verwenden. Es sind auch enzymatische Varianten zur Einführung von chemisch aktivierten Positionen an eine Ziel-DNA beschrieben worden. Hier wird an einer Ziel-DNA enzymatisch eine 5'-NH₂-Funktionalisierung durchgeführt.

Für die Abtastung eines immobilisierten DNA-Arrays sind vielfach fluoreszent markierte Sonden verwendet worden. Besonders geeignet für Fluoreszenzmarkierungen ist das einfache Anbringen von Cy3 und Cy5 Farbstoffen am 5'-OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Neuere Verfahren zum Nachweis von Mutationen sind im folgenden aufgeführt:
Als ein Spezialfall der Sequenzierung ist die Einzelbasen-Primer-Erweiterung (Genetic Bit Analysis) erwähnenswert (Head, SR., Rogers, YH., Parikh K., Lan, G., Anderson, S., Goelet, P., Boycejacino MT., Nucleic Acids Research. 25(24): 5065-5071, 1997; Picoult-Newberg, L., Genome Res. 9(2): 167-174, 1999). Eine kombinierte Amplifikation und Sequenzierung wird in US-A1 5928906 beschrieben, wo eine basenspezifische Terminierung auf Matrixmolekülen eingesetzt wird. Ein weiteres Verfahren setzt eine Ligase/Polymerasereaktion für die Identifikation von Nukleotiden ein (US-A1 5952174).

Matrix-assistierte Laser Desorptions/Ionisations-Massenspektrometrie (MALDI) ist eine sehr leistungsfähige Entwicklung für die Analyse von Biomolekülen (Karas, M. und Hillenkamp, F. (1988), Laser desorption ionization of proteins with molecular masses exeeding 10000 daltons. Anal. Chem. 60: 2299-2301). Ein Analyt wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyten erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

Maldi eignet sich ausgezeichnet zur Analyse von Peptiden und Proteinen. Die Analyse von Nukleinsäuren ist etwas schwieriger (Gut, I. G. und Beck, S. (1995), DNA and Matrix Assisted Laser Desorption Ionization Mass Spectrometry. Molecular Biology: Current Innovations and Future Trends 1: 147-157.) Für Nucleinsäuren ist die Empfindlichkeit etwa 100 mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. Für MALDI spielt die Wahl der Matrix eine eminent wichtige Rolle. Für die Desorption von Peptiden sind einige sehr leistungsfähige Matrices gefunden worden, die eine sehr feine Kristallisation ergeben. Für DNA gibt es zwar mittlererweile einige ansprechende Matrices, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I. G. und Beck, S. (1995), A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit um den gleichen Betrag, wie er für Peptide gefunden wird. Ein weiterer Vorteil von "charge tagging" ist die erhöhte Stabilität der Analyse gegen Verunreinigungen, die den Nachweis unmodifizierter Substrate stark erschweren.

Genomische DNA wird durch Standardmethoden aus DNA von Zell-, Gewebe- oder sonstigen Versuchsproben gewonnen. Diese Standardmethodik findet sich in Referenzen wie Fritsch und Maniatis eds., Molecular Cloning: A Laboratory Manual, 1989.

Gemeinsamkeiten zwischen Promotoren bestehen nicht nur im Vorkommen von TATA- oder GC-Boxen sondern auch darin, für welche Transkriptionsfaktoren sie Bindestellen besitzen und in welchem Abstand diese sich zueinander befinden. Die existierenden Bindestellen für ein bestimmtes Protein stimmen in ihrer Sequenz nicht vollständig überein, es finden sich aber konservierte Folgen von mindestens 4 Basen, die durch das Einfügen von "Wobbles", d. h. Positionen, an denen sich jeweils unterschiedliche Basen befinden, noch verlängert werden können. Des weiteren liegen diese Bindestellen in bestimmten Abständen zueinander vor.

Die Verteilung der DNA im Interphase-Chromatin, das den größten Teil des nuklearen Volumens einnimmt, unterliegt jedoch einer ganz speziellen Ordnung. So ist die DNA an mehreren Stellen an die nukleare Matrix, eine filamentöse Struktur an der Innenseite der nuklearen Membran, angeheftet. Diese Regionen bezeichnet man als matrix attachment regions (MAR) oder scaffold attachment regions (SAR). Das Anheften hat wesentlichen Einfluß auf die Transkription bzw. die Replikation. Diese MAR-Fragmente weisen keine konservativen Sequenzen auf, bestehen allerdings zu 70% aus A bzw. T und liegen in der Nähe von cisagierenden Regionen, die die Transkription allgemein regulieren, und Topoisomerase II-Erkennungsstellen.

Neben Promotoren und Enhancern existieren weitere regulatorische Elemente für verschiedene Gene, sogenannte Insulators. Diese Insulators können z.B. die Wirkung des Enhancers auf den Promotor inhibieren, wenn sie zwischen Enhancer und Promotor liegen, oder aber, zwischen Heterochromatin und einem Gen gelegen, das aktive Gen vor dem Einfluß des Heterochromatins schützen. Beispiele für solche Insulators sind: 1. sogenannte LCR (locus control regions), welche aus mehreren gegenüber DNAase I hypersensitiven Stellen besteht; 2. bestimmte Sequenzen wie SCS (specialized chromatin structures) bzw. SCS', 350 bzw. 200 bp lang und hoch-resistent gegen Degradierung durch DNAase I und auf beiden Seiten von hypersensitiven Stellen flankiert (Abstand je 100 bp). An scs' bindet das Protein BEAF-32. Diese Insulators können auf beiden Seiten des Gens liegen.

In der WO 98 56952 A (Univ. Southern California)wird ein Krebs Diagnoseverfahren beschrieben, das auf einer Primer-Extension und unterschiedlichen Methylierungen der DNA basiert.

Cheryl L. Paul und Susan J. Clark ("Cytosine Methylation: Quantitation by Automated Genomic Sequencing and GENESCAN™ Analysis", BioTechniques, 1996, Vol. 21, Nr. 1, Seiten 126-133) beschreiben die Bisulfitbehandlung, PCR-Amplifikation und Sequenzierung von genomischer DNA.

Ferner beschreiben Christof M. Niemeyer und Dietmar Blohm ("DNA Microarrays", Angewandte Chemie Int. Ed., Wiley-VCH Verlag GmbH, 1999, Bd. 38, Nr. 19, Seiten 2865-2869) massenspektrometrische Nachweismethoden von SNPs unter Verwendung des MALDI-MS.

In der US 5,605,798 werden auf Massenspektrometrie basierende Verfahren zur Detektion von Nukleinsäuresequenzen beschrieben.

Aufgabe der vorliegenden Erfindung ist es ein Verfahren bereitstellen, welches sich zum gleichzeitigen Detektieren von Cytosin-Methylierungen und SNPs in genomischen DNA-Proben besonders eignet. Dabei soll bevorzugt eine Vielzahl von Fragmenten gleichzeitig untersucht werden können.

Die Aufgabe wird erfindungsgemäß durch ein Verfahren zum Nachweis von 5-Methylcytosin in genomischen DNA-Proben gelöst, wobei man die folgenden Schritte ausführt:
(a) man setzt eine genomische DNA aus einer DNA-Probe mit einem Reagenz chemisch um, wobei 5-Methylcytosin und Cytosin unterschiedlich reagieren und diese somit nach der Reaktion ein unterschiedliches Basenpaarungsverhalten in der DNA Duplex zeigen;
(b) man amplifiziert vorbehandelte DNA unter Verwendung einer Polymerase und mindestens einem Oligonukleotid (Typ A) als Primer;
(c) man hybridisiert die amplifizierte genomische DNA an mindestens ein Oligonukleotid (Typ B) mit bekannter Sequenz von n Nucleotiden unter Ausbildung einer Duplex, wobei besagte hybridisierte Oligonukleotide des Typs B mit ihrem 3'-Ende teilweise oder vollständig an die Positionen hybridisieren, die hinsichtlich ihrer Methylierung in der genomischen DNA-Probe zu untersuchen sind;
(d) man verlängert das Oligonukleotid (Typ B), sofern es mit seinem 3'Terminus zuvor ohne Basenfehlpaarungen an der zu untersuchenden Position hybridisierte, mittels ei-ner Polymerase mindestens um ein Nukleotid, wobei mindestens ein Nukleotid eine nachweisbare Markierung trägt und die Verlängerung vom Methylierungsstatus des jeweiligen Cytosins in der genomischen DNA-Probe abhängt;
(e) man untersucht die verlängerten Oligonukleotide werden auf das Vorhandensein der Markierung.

Erfindungsgemäß bevorzugt ist es, dass man die Oligonukleotide (Typ B) an definierten Stellen an eine Festphase bindet oder dass man die Amplifikate an definierten Stellen an eine Festphase bindet.

Bevorzugt ist es ferner, dass man unterschiedliche Oligonukleotidsequenzen auf einer ebenen Festphase in Form eines rechtwinkligen oder hexagonalen Gitters anordnet.

Weietrhin ist es erfindungsgemäß bevorzugt, dass die Festphasenoberfläche aus Silizium, Glas, Polystyrol, Aluminium, Stahl, Eisen, Kupfer, Nickel, Silber oder Gold besteht.

Bevorzugt ist auch, dass die an den verlängerten Oligonukleotiden angebrachten Markierungen an jeder Position der Festphase, an der sich eine Oligonukleotidsequenz befindet, identifizierbar sind.

Es ist erfindungsgemäß bevorzugt, dass bei der Amplifikation mindestens ein Primer (Typ A) an eine Festphase gebunden ist.

Außerdem kann es erfindungsgemäß bevorzugt sein, dass man unterschiedliche Amplifikate auf der Festphase in Form eines rechtwinkligen oder hexagonalen Gitters anordnet.

Ganz besonders bevorzugt ist es, dass man die Behandlung der DNA vor der Amplifikation mit einer Bisulfitlösung (=Disulfit, Hydrogensulfit) durchführt.

Insbesondere ist es erfindungsgemäß bevorzugt, dass die Amplifikation mittels der Polymerasekettenreaktion (PCR) erfolgt.

Es ist ferner erfindungsgemäß besonders bevorzugt dass die verwendeten Oligonukleotide des Typs A entweder nur die Basen T, A und C oder aber die Basen T, A und G enthalten.

Bevorzugt ist es ferner, dass die Markierungen der Nukleotide Fluoreszenzmarkierungen sind.

Weiterhin ist es erfindungsgemäß bevorzugt, dass die Markierungen der Nukleotide Radionuklide sind.

Besonders bevorzugt ist es, dass die Markierungen der Nukleotide ablösbare Massenmarkierungen sind, die in einem Massenspektrometer nachgewiesen werden.

Weietrhin ist es erfindungsgemäß bevorzugt, dass man die verlängerten Oligonukleotide insgesamt im Massenspektrometer nachweist und somit durch ihre Masse eindeutig markiert sind. Besonders bevorzugt ist es auch, dass man jeweils ein Fragment der verlängerten Oligonukleotide im Massenspektrometer nachweist.

Es ist weiterhin erfindungsgemäß bevorzugt, dass man das Fragment des verlängerten Oligonukleotids durch Verdau mit einer oder mehrerer Exo- oder Endonukleasen erzeugt.

Erfindungsgemäß bevorzugt ist es ferner, dass zur besseren Detektierbarkeit im Massenspektrometer die erzeugten Fragmente eine einzelne positive oder negative Nettoladung aufweisen.

Ganz besonders ist es erfindungsgemäß bevorzugt, dass man die Detektion der verlängerten Oligonukleotide mittels Matrix assistierter Laser Desorptions/Ionisations Massenspektrometrie (MALDI) oder mittels Elektrospray Massenspektrometrie (ESI) durchführt und visualisiert.

Es ist weiterhin erfindungsgemäß ein Verfahren bevorzugt, wobei die Polymerasen hitzebeständige DNA-Polymerasen sind.

Bevorzugt ist erfindungsgemäß auch ein Verfahren, wobei man zusätzlich zur DNA-Methylierung auch SNPs detektiert und visualisiert.

Es ist ferner ein Verfahren bevorzugt, wobei die eingesetzten Nukleotide terminierende (Typ C 2) und/oder kettenverlängernde Nukleotide (Typ C 1) sind.

Erfindungsgemäß bevorzugt ist ferner ein Verfahren, wobei man die Nukleotide (Typ C 1 und C2) aus einer Gruppe auswählt, die entweder die Nukleobasen A, T und C oder aber die Basen G und A und T enthält.

Weiterhin ist es erfindungsgemäß bevorzugt, dass man die Amplifikation von mehreren DNA-Abschnitten in einem Reaktionsgefäß durchführt.

Ganz besonders ist ein Verfahren erfindungsgemäß bevorzugt, wobei die genomische DNA aus einer DNA-Probe erhalten wurde, wobei Quellen für DNA z. B. Zelllinien, Blut, Sputum, Stuhl, Urin, Gehirn-Rückenmarks-Flüssigkeit, in Paraffin einbettetes Gewebe, histologische Objektträger und alle möglichen Kombinationen hiervon umfassen.

Schließlich ist es erfindungsgemäß bevorzugt, dass man Methylierungsanalysen des oberen und unteren DNA-Stranges in einem Experiment durchführt. Die Durchführung der Analysen erfolgt bevorzugt gleichzeitig.

Beschrieben wird also ein Verfahren zum Nachweis von Methylcytosin in genomischen DNA-Proben:
Die Methode beinhaltet die Amplifikation, Hybridisierung und Verlängerungsreaktion einer gesamten DNA oder eines Fragments hiervon. Die Methode kann benutzt werden zum Nachweis von Methylcytosin und auch gleichzeitig von Single Nucleotide Polymorphisms (SNPs) und Mutationen.
Die zu analysierende genomische DNA wird bevorzugt aus üblichen Quellen für DNA erhalten, wie z. B. Zelllinien, Blut, Sputum, Stuhl, Urin, Gehirn-Rückenmarks-Flüssigkeit, in Paraffin einbettetes Gewebe, histologische Objektträger und alle möglichen Kombinationen hiervon.

Im ersten Schritt des Verfahrens wird die eingesetzte DNA bevorzugt mit Bisulfit, (= Disulfit, Hydrogensulfit) oder aber einer anderen Chemikalie derart behandelt, dass alle nicht an der 5-Position der Base methylierten Cytosinbasen so verändert werden, dass eine hinsichtlich dem Basenpaarungsverhalten unterschiedliche Base entsteht, während die in 5-Position methylierten Cytosine unverändert bleiben. Wird Bisulfit verwendet, so findet an den nicht methylierten Cytosinbasen eine Addition statt. Die anschließende alkalische Hydrolyse führt dann zur Umwandlung von nicht methylierten Cytosin-Nukleobasen in Uracil.

Im zweiten Schritt des Verfahrens wird die vorbehandelte DNA bevorzugt unter Verwendung einer hitzebeständigen Polymerase und mindestens einem Primer (Typ A) amplifiziert.

In einer besonders bevorzugten Variante des Verfahrens wird die Amplifikation mit Primern des Typs A mittels der Polymerasekettenreaktion (PCR) durchgeführt.

In einer bevorzugten Variante des Verfahrens wird die Amplifikation von mehreren DNA-Fragmenten in einem Reaktionsgefäß durchgeführt. Dies kann entweder eine sogenannte Multiplex PCR sein, in der verschiedene Primer jeweils definierte Fragmente erzeugen. Es werden verschiedene definierte Amplifikationen in einem Reaktionsgefäß durchgeführt. In einer weiteren, besonders bevorzugten Variante des Verfahrens amplifizieren Primer gezielt und reproduzierbar jeweils mehrere Fragmente. Dies kann entweder dadurch erzielt werden, dass sie beispielsweise an repetitive Elemente im Genom binden. In einer besonders bevorzugten Variante des Verfahrens binden die Primer an Transcription Factor Binding Sites, an Promotoren oder andere regulatorische Elemente in Genen. In einer besonders bevorzugten Variante des Verfahrens findet die Amplifikation durch Verlängerung von Primers statt, die an eine Festphase gebunden sind. Eine Multiplex-PCR im weiteren Sinne kann dadurch ausgeführt werden, dass unterschiedliche Primer an verschiedenen, definierten Orten einer Festphase gebunden sind.

In einer wiederum bevorzugten Variante des zweiten Verfahrensschrittes ist die Festphase eben, wobei die unterschiedlichen Oligonukleotidsequenzen in Form eines rechtwinkligen oder hexagonalen Gitters angeordnet sind. Das hat zur Folge, dass auch die unterschiedlichen Amplifikate auf der Festphase in Form eines rechtwinkligen oder hexagonalen Gitters angeordnet sind. Wie bereits oben beschrieben, werden in diesem Fall mehrere Amplifikate direkt auf der Festphase erzeugt.

Die Festphasenoberfläche besteht bevorzugt aus Silizium, Glas, Polystyrol, Aluminium, Stahl, Eisen, Kupfer, Nickel, Silber oder Gold.

In einer besonders bevorzugten Variante des Verfahrens enthalten die Oligonukleotide des Typs A entweder nur die Basen T, A und C oder nur die Basen T, A und G.

Im dritten Verfahrensschritt wird die amplifizierte genomische DNA an mindestens einen Primer (Typ B) unter Ausbildung einer Duplex hybridisiert. Die hybridisierten Oligonukleotide des Typs B binden jeweils an ihrem 3'-Ende an die Positionen, die hinsichtlich ihrer Methylierung in der genomischen DNA-Probe zu untersuchen sind. Dabei können zwei Fälle unterschieden werden: entweder ist die zu untersuchende Sequenz zu dem Primer auch an seinem 3'-Ende vollständig komplementär, in diesem Fall ist eine Verlängerung des Primers im nächsten Schritt in einer Polymerasereaktion möglich, oder aber die Sequenz ist zu der des Primers am 3'-Ende nicht vollständig komplementär, in diesem Fall kann keine Verlängerung des Primers erfolgen. Ist eine bestimmte CpG Position auf Methylierung hin zu untersuchen, so gibt es zwei mögliche Zustände. Nach der chemischen Vorbehandlung, bevorzugt mit Bisulfit, ergibt sich bei Methylierung ein CG, bei Vorliegen eines unmethylierten Cytosins ein UG bzw. nach der Amplifikation ein TG. Bevorzugt wird das Experiment in diesem Fall mit zwei unterschiedlichen Primern durchgeführt, die jeweils für einen der Zustände eine vollständige Komplementarität und damit die Möglichkeit zur Kettenverlängerung in jeweils einem der beiden möglichen Fälle ergeben.

Sind nicht bereits die Amplifikate an eine Festphase gebunden, so können die an die Amplifikate hybridisierten Oligonukleotide mit ihrem 5'-Ende oder an einer anderen Base oder über ihr Rückgrat mit einer Festphase verbunden sein, nicht aber über ihr 3'-Ende. Bevorzugt erfolgt eine Bindung über das 5'-Ende. In einer bevorzugten Variante ist die Festphase eben, wobei die unterschiedlichen Oligonukleotidsequenzen (Typ B) in Form eines rechtwinkligen oder hexagonalen Gitters angeordnet sind.

Die Festphasenoberfläche besteht bevorzugt aus Silizium, Glas, Polystyrol, Aluminium, Stahl, Eisen, Kupfer, Nickel, Silber oder Gold.

Im vierten Verfahrensschritt wird das entstandene Oligonukleotid mit einer hitzebeständigen Polymerase um mindestens ein bis maximal zehn Nukleotide verlängert, wobei zumindest ein Nukleotid eine nachweisbare Markierung trägt. Die Art der Verlängerung hängt dabei vom Methylierungsstatus des jeweiligen Cytosins in der genomischen DNA-Probe ab, oder aber auch von eventuell vorhandenen SNPs, Punktmutationen oder Deletionen, Insertionen und Inversionen.

Prinzipiell sind nur terminierende Oligonuleotide (Typ C2) erforderlich. Je nach Sequenz können jedoch auch kettenverlängernde Oligonukleotide verwendet werden, sofern es im jeweiligen Sequenzkontext möglich ist.

In einer bevorzugten Variante des Verfahrens sind die eingesetzten Nukleotide terminierende (Typ C2) und/oder kettenverlängernde Nukleotide (Typ C1). In einer besonders bevorzugten Variante des Verfahrens werden die Nukleobasen des Typs C1 und/oder des Typs C2 aus einer Gruppe ausgewählt, die die Basen T, A und C oder aber die Basen T, A und G enthält.

Die Markierung der verlängerten Oligonukleotide des Typs B erfolgt bevorzugt über absorbierende Farbstoffe und/oder über Chemilumineszenz und/oder über radioaktive Isotope und/oder über Fluoreszenzmarkierungen, die über die im vierten Verfahrensschritt angefügten Nukleotide eingeführt werden. Bevorzugt ist ebenfalls die Markierung über die Molekülmasse des verlängerten Oligonukleotids. Vorzugsweise wird der Fluoreszenzmarker durch ein fluoreszenzmarkiertes Nukleotid wie z. B. Cy5-dCTP eingeführt.

Im fünften Verfahrensschritt werden die verlängerten Oligonukleotide auf das Vorhandensein einer Markierung untersucht. Wird eine ebene Festphase verwendet, so erfolgt eine Analyse an jedem Ort auf der Festphase, an dem ursprünglich ein Oligonukleotid immobilisiert wurde.

In einer besonders bevorzugten Variante des Verfahrens erfolgt der Nachweis der verlängerten Oligonukleotide über ihre Fluoreszenz.

In einer bevorzugten Variante des Verfahrens werden Fragmente des verlängerten Oligonukleotids durch Verdau mit einer oder mehrerer Exo- oder Endonukleasen erzeugt.

In einer besonders bevorzugten Variante des Verfahrens sind die Markierungen der Nukleotide ablösbare Massenmarkierungen, die in einem Massenspektrometer nachweisbar sind.

Ablösbare Massenmarkierungen, die verlängerten Oligonukleotide insgesamt oder Fragmente hiervon werden in einer besonders bevorzugten Variante des Verfahrens mittels Matrix-assistierter Laser-Desorptions/Ionisations-Massenspektrometrie (MALDI-MS) oder mittels Elektronenspray-Massenspektrometrie (ESI) anhand ihrer eindeutigen Masse nachgewiesen und visualisiert.

Vorzugsweise weisen die im Massenspektrometer detektierten Fragmente eine einzelne positive oder negative Nettoladung auf.

In einer besonders bevorzugten Variante des Verfahrens werden SNPs (Single Nucleotide Polymorphisms) und Cytosin-Methylierungen in einem Experiment analysiert.

In einer besonders bevorzugten Variante des Verfahrens werden der untere und der obere Strang der DNA-Probe nach der chemischen Vorbehandlung in einem Experiment analysiert, um eine interne experimentelle Kontrolle sicherzustellen.

Weiterer Gegenstand der Erfindung ist ein Kit, das Chemikalien und Hilfsmittel zur Durchführung der Bisulfit-Reaktion und/oder der Amplifikation, der Hybridisierung, der Verlängerungsreaktion und/oder Polymerasen und/oder die Dokumentation zur Durchführung des Verfahrens enthält.

Die folgenden Beispiele erläutern die Erfindung:

### Beispiel 1:

Das folgende Beispiel bezieht sich auf ein Fragment von Exon 23 des Faktor VIII-Gens, in dem eine bestimmte CG-Position auf Methylierung zu untersuchen ist.

Im ersten Schritt wird das Fragment durch Primer des Typs A amplifiziert und zwar durch ATTATGTTGGAGTAGTAGAGTTTAAATGGTT (SEQ-ID No.: 1) und ACTTAACACTTACTATTTAAATCACAACCCAT (SEQ-ID No.: 2). Die amplifizierte DNA wird an ein Oligonukleotid des Typs B (beispielsweise GTTGGATGTTGTTGAGAAACG (SEQ-ID No.: 3)) hybridisiert und in einer Polymerasereaktion mit einem markierten 2',3'-Didesoxythymidintriphosphat (Typ C2) verlängert. Nur wenn ein CG, das heißt in der ursprünglichen genomischen DNA-Probe ein methyliertes Cytosin vorgelegen hat, kann diese Verlängerung erfolgen, da sonst eine Fehlpaarung am 3'-Ende des Primers die Polymerasereaktion verhindert. Somit entscheidet der Methylierungsstatus des jeweiligen zu untersuchenden Cytosins über die Verlängerung des Primers.

Zur Kontrolle kann die Reaktion mit dem Primer GTTGGATGTTGTTGAGAAATG (SEQ-ID No.: 4) durchgeführt werden. In diesem Fall findet die Verlängerung nur dann statt, wenn in der zu untersuchenden DNA-Probe an besagter Position ein unmethyliertes Cytosin vorgelegen hat. Die Label können beispielsweise absorbierende Farbstoffe wie Megaprime^{™} für ddTTP oder Rediprime 11^{™} sein.

### Beispiel 2:

Das folgende Beispiel bezieht sich auf ein Fragment von Exon 23 des Faktor VIII-Gens, in dem eine bestimmte CG-Position auf Methylierung zu untersuchen ist.

Im ersten Schritt wird das Fragment durch Primer des Typs A amplifiziert und zwar durch ATTATGTTGGAGTAGTAGAGTTTAAATGGTT (SEQ-ID No.: 1) und ACTTAACACTTACTATTTAAATCACAACCCAT (SEQ-ID No.: 2). Die amplifizierte DNA wird an ein Oligonukleotid des Typs B (beispielsweise GTTGGATGTTGTTGAGAAACG (SEQ-ID No.: 3)) hybridisiert, das mit seinem 5'-Ende an eine Festphasenoberfläche immobilisiert ist, und in einer Polymerasereaktion mit einem markierten 2',3'-Didesoxythymidintriphosphat (Typ C2) verlängert. Nur wenn ein CG, das heißt in der ursprünglichen genomischen DNA-Probe ein methyliertes Cytosin vorgelegen hat, kann diese Verlängerung erfolgen, da sonst eine Fehlpaarung am 3'-Ende des Primers die Polymerasereaktion verhindert. Somit entscheidet der Methylierungsstatus des jeweiligen zu untersuchenden Cytosins über die Verlängerung des Primers.

Zur Kontrolle kann die Reaktion mit dem Primer GTTGGATGTTGTTGAGAAATG (SEQ-ID No.: 4) durchgeführt werden. In diesem Fall findet die Verlängerung nur dann statt, wenn in der zu untersuchenden DNA-Probe an besagter Position ein unmethyliertes Cytosin vorgelegen hat. Die Label können beispielsweise absorbierende Farbstoffe wie Megaprime^{™} für ddTTP oder Rediprime II^{™} sein.

### Beispiel 3:

Das folgende Beispiel bezieht sich auf ein Fragment von Exon 23 des Faktor VIII-Gens, in dem eine bestimmte CG-Position auf Methylierung zu untersuchen ist.

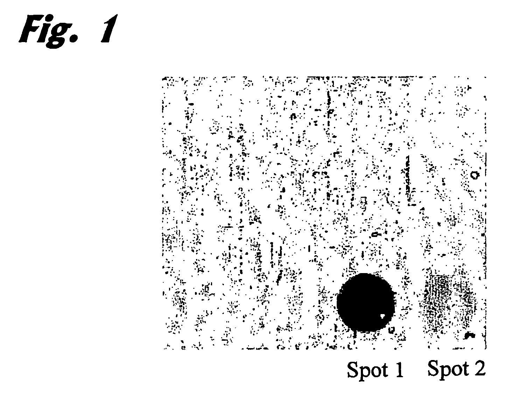

Im ersten Schritt wird das Fragment durch Primer des Typs A amplifiziert und zwar durch ATTATGTTGGAGTAGTAGAGTTTAAATGGTT (SEQ-ID No.: 1) und ACTTAACACTTACTATTTAAATCACAACCCAT (SEQ-ID No.: 2). Für diese Amplifikation wurde mit Bisulphit behandelte DNA für 5 min bei 96 °C inkubiert und dann in 40 Zyclen jeweils 55 sec bei 96 °C denaturiert, 75 sec bei 61.7 °C inkubiert (Annealing) und 100 sec bei 72 °C inkubiert (E-longation). In einer anschließenden Reaktion (final Extension) wird der Reaktionsansatz 15 min bei 72 °C inkubiert. Die amplifizierte DNA wird an die Oligonukleotide AAAAACTACAAAAACTCT (SEQ-ID No.: 5) (Spot 1 in Fig. 1) und AAAAACTACGAAAACTCT (SEQ-ID No.: 6) (Spot 2 in Fig. 1) hybridisiert, die an einer festen Phase immobilisiert sind. Für die Verlängerungsreaktion wird 2'-Desoxythymidintriphosphat (dTTP, als Typ C1), 2'-Desoxyguanosintriphosphat (dGTP, als Typ C1), 2'-Desoxyadenosintriphosphat (dATP, als Typ C1) und 2'-Desoxycytidintriphosphat (dCTP, als Typ C1) benötigt, wobei zusätzlich fluoreszenzmarkiertes 2'-Desoxycytidintriphosphat im Verhältnis von 3:1 (bezogen auf nicht markiertes 2'-Desoxycytidintriphosphat) zugegeben wird. Der Reaktionsansatz, bestehend aus dem Amplifikat, dem Desoxynukleotidgemisch und 10xPuffer, wird für 15 min bei 96 °C inkubiert. Für die anschließende Primer-Extension-Reaktion wird eine DNA-Polymerase, hier Klenow Fragment, zugesetzt und das Reaktionsgemisch über Nacht bei 37 °C auf der festen Phase inkubiert . Wie in der folgenden Abbildung zu erkennen, läßt die durchgeführte Primer Extension auf den Methylierungsstatus der DNA schließen. Ein Vergleich von Spot 1 und Spot 2, wie in Fig. 1 dargestellt, erlaubt eine Aussage über den Methylierungsstatus: im vorliegenden Fall wird durch die Stärke des Signals von Spot 1 ein nicht methyliertes CpG nachgewiesen.

## Patentansprüche

1. Verfahren zum Nachweis von 5-Methylcytosin oder zum Nachweis von 5-Methylcytosin und SNP in genomischen DNA-Proben, **dadurch gekennzeichnet, dass** man die folgenden Schritte ausführt:
(a) man setzt eine genomische DNA aus einer DNA-Probe mit einem Reagenz chemisch um, wobei 5-Methylcytosin und Cytosin unterschiedlich reagieren und diese somit nach der Reaktion ein unterschiedliches Basenpaarühgsverhalten in der DNA Duplex zeigen;
(b) man amplifiziert vorbehandelte DNA unter Verwendung einer Polymerase und mindestens einem Oligonukleotidprimer;
(c) man hybridisiert die amplifizierte genomische DNA unter stringenten Bedingungen an mindestens ein Oligonukleotid mit bekannter Sequenz, wobei es mindestens zwischen einer Base im 3' Bereich des Oligonukleotids und der in der DNA zu untersuchenden Base je nach Methylierungsstatus der Ursprungs-DNA entweder zu einer Fehlpaarung oder zu keiner Fehlpaarung kommt;
(d) man verlängert die Oligonukleotide, die im 3' Bereich mit der in der DNA zu untersuchenden Base keine Fehlpaarung aufweisen, mittels einer Polymerase mindestens um ein Nukleotid, wobei mindestens ein Nukleotid eine nachweisbare Markierung trägt, während die Oligonukleotide, die im 3' Bereich mit der zu untersuchenden Base eine Fehlpaarung aufweisen, nicht verlängert werden;
(e) man untersucht die verlängerten Oligonukleotide auf das Vorhandensein der Markierung.

2. Verfahren nach Anspruch 1, **dadurch gekennzeichnet, dass** man das Oligonukleotid oder die Oligonukleotide gemäß Anspruch 1 c an definierten Stellen an eine Festphase bindet.

3. Verfahren nach Anspruch 1, **dadurch gekennzeichnet, dass** man die Amplifikate an definierten Stellen an eine Festphase bindet.

4. Verfahren nach Anspruch 2, **dadurch gekennzeichnet, dass** man unterschiedliche Oligonukleotidsequenzen auf einer ebenen Festphase in Form eines rechtwinkligen oder hexagonalen Gitters anordnet.

5. Verfahren nach einem der Ansprüche 2 bis 4, **dadurch gekennzeichnet, dass** die Festphasenoberfläche aus Silizium, Glas, Polystyrol, Aluminium, Stahl, Eisen, Kupfer, Nickel, Silber oder Gold besteht.

6. Verfahren nach einem der Ansprüche 2 bis 5, **dadurch gekennzeichnet, dass** die an den verlängerten Oligonukleotiden angebrachten Markierungen an jeder Position der Festphase, an der sich eine Oligonukleotidsequenz befindet, identifizierbar sind.

7. Verfahren nach Anspruch 1, **dadurch gekennzeichnet, dass** bei der Amplifikation mindestens ein Primer, der nicht identisch ist mit dem Oligonukleotid aus Anspruch 1 b, an definierten Stellen an eine Festphase bindet.

8. Verfahren nach Anspruch 1, 3 oder 7, **dadurch gekennzeichnet, dass** man unterschiedliche Amplifikate auf der Festphase in Form eines rechtwinkligen oder hexagonalen Gitters anordnet.

9. Verfahren nach einem der voranstehenden Ansprüche, **dadurch gekennzeichnet, dass** man die Behandlung der DNA vor der Amplifikation mit einer Bisulfitlösung (=Disulfit, Hydrogensulfit) durchführt.

10. Verfahren nach einem der voranstehenden Ansprüche, **dadurch gekennzeichnet, dass** die Amplifikation mittels der Polymerasekettenreaktion (PCR) erfolgt.

11. Verfahren nach einem der vorangehenden Ansprüche, **dadurch gekennzeichnet dass** die in Anspruch 1 verwendeten und in Anspruch 7 spezifizierten Oligonukleotide entweder nur die Basen T, A und C oder aber die Basen T, A und G enthalten.

12. Verfahren nach einem der voranstehenden Ansprüche, **dadurch gekennzeichnet, dass** die Markierungen der Nukleotide Fluoreszenzmarkierungen sind.

13. Verfahren nach einem der Ansprüche 1 bis 11, **dadurch gekennzeichnet, dass** die Markierungen der Nukleotide Radionuklide sind.

14. Verfahren nach einem der Ansprüche 1 bis 11, **dadurch gekennzeichnet, dass** die Markierungen der Nukleotide ablösbare Massenmarkierungen sind, die in einem Massenspektrometer nachgewiesen werden.

15. Verfahren nach einem der Ansprüche 1 bis 11, **dadurch gekennzeichnet, dass** man die verlängerten Oligonukleotide insgesamt im Massenspektrometer nachweist und somit durch ihre Masse eindeutig markiert sind.

16. Verfahren nach einem der Ansprüche 1 bis 11, **dadurch gekennzeichnet, dass man jeweils ein Fragment der** verlängerten Oligonukleotide im Massenspektrometer nachweist.

17. Verfahren nach einem des Anspruch 1 bis 16, **dadurch gekennzeichnet, dass** zur besseren Detektierbarkeit im Massenspektrometer die erzeugten Fragmente eine einzelne positive oder negative Nettoladung aufweisen.

18. Verfahren gemäß einem der voranstehenden Ansprüche, **dadurch gekennzeichnet, dass** man die Detektion der verlängerten Oligonukleotide mittels Matrix assistierter Laser Desorptions/Ionisations Massenspektrometrie (MALDI) oder mittels Elektrospray Massenspektrometrie (ESI) durchführt und visualisiert.

19. Verfahren nach einem der voranstehenden Ansprüche, wobei die Polymerasen hitzebeständige DNA-Polymerasen sind.

20. Verfahren nach einem der voranstehenden Ansprüche, wobei man zusätzlich zur DNA-Methylierung auch SNPs detektiert und visualisiert.

21. Verfahren nach einem der voranstehenden Ansprüche, wobei die eingesetzten Nukleotide terminierende und/oder kettenverlängernde Nukleotide sind.

22. Verfahren nach einem der voranstehenden Ansprüche, wobei man die Nukleotide aus einer Gruppe auswählt, die entweder die Nukleobasen A, T und C oder aber die Basen G und A und T enthält.

23. Verfahren nach Anspruch 1, wobei die genomische DNA aus einer DNA-Probe erhalten wurde, wobei Quellen für DNA z. B. Zelllinien, Blut, Sputum, Stuhl, Urin, Gehirn-Rückenmarks-Flüssigkeit, in Paraffin einbettetes Gewebe, histologische Objektträger und alle möglichen Kombinationen hiervon umfassen.

24. Verfahren nach einem der vorangehenden Ansprüche, **dadurch gekennzeichnet, dass** man Methylierungsanalysen der beiden DNA-Strange in einem Experiment gleichzeitig durchführt.

## Claims

1. A method for detecting 5-methylcytosine or detecting 5-methylcytosine and SNP in genomic DNA samples, **characterized in that** the following steps are carried out:
(a) a genomic DNA from a DNA sample is chemically converted with a reagent, 5-methylcytosine and cytosine reacting differently, thus exhibiting different base pairing behaviors in the DNA duplex subsequent to the reaction;
(b) pretreated DNA is amplified using a polymerase and at least one oligonucleotide primer;
(c) the amplified genomic DNA is hybridized under stringent conditions to at least one oligonucleotide having a known sequence, whereby between at least one base in the 3' region of the oligonucleotide and the base in the DNA to be investigated, according to the status of methylation of the original DNA, either a mispairing or else no mispairing occurs;
(d) the oligonucleotides having no mispairing with the base to be investigated in the 3' region of the DNA, are elongated by at least one nucleotide by means of a polymerase, whereby at least one nucleotide is carrying a detectable label, whereas the oligonucleotides having a mispairing with the base to be investigated in the 3' region, are not elongated;
(e) the elongated oligonucleotides are analyzed for the presence of the label.

2. The method as recited in claim 1, **characterized in that** the oligonucleotide or the oligonucleotides according to claim 1 c are bonded to a solid phase at defined locations.

3. The method as recited in claim 1, **characterized in that** the amplificates are bonded to a solid phase at defined locations.

4. The method as recited in claim 2, **characterized in that** different oligonucleotide sequences are arranged on a plane solid phase in the form of a rectangular or hexagonal lattice.

5. The method as recited in one of the claims 2 through 4, **characterized in that** solid phase surface is composed of silicon, glass, polystyrene, aluminum, steel, iron, copper, nickel, silver, or gold.

6. The method as recited in one of the claims 2 through 5, **characterized in that** the labels attached to the elongated oligonucleotides are identifiable at each position of the solid phase at which an oligonucleotide sequence is located.

7. The method as recited in claim 1, **characterized in that** at least one primer that is not identical with the oligonucleotide of claim 1 b is bonded to a solid phase during amplification.

8. The method as recited in claim 1, 3, or 7, **characterized in that** different amplificates are arranged on the solid phase in the form of a rectangular or hexagonal lattice.

9. The method as recited in one of the preceding claims, **characterized in that**, prior to the amplification, the DNA is treated with a bisulfite solution (=disulfite, hydrogen sulfite).

10. The method as recited in one of the preceding claims, **characterized in that** the amplification is carried out by means of the polymerase chain reaction (PCR).

11. The method as recited in one of the preceding claims, **characterized in that** the oligonucleotides used in Claim 1 and specified in claim 7 either contain only the bases T, A and C or else the bases T, A und G.

12. The method as recited in one of the preceding claims, **characterized in that** the labels of the nucleotides are fluorescence labels.

13. The method as recited in one of the claims 1 through 11, **characterized in that** the labels of the nucleotides are radionuclides.

14. The method as recited in one of the claims 1 through 11, **characterized in that** the labels of the nucleotides are detachable mass labels which are detected in a mass spectrometer.

15. The method as recited in one of the claims 1 through 11, **characterized in that** the elongated oligonucleotides altogether are detected in the mass spectrometer, thus being uniquely labeled by their masses.

16. The method as recited in one of the claims 1 through 11, **characterized in that** in each case one fragment of the elongated oligonucleotides is detected in the mass spectrometer.

17. The method as recited in one of the claims 1 through 16, **characterized in that** the produced fragments have a single positive or negative net charge for better detectability in the mass spectrometer.

18. The method as recited in one of the preceding claims, **characterized in that** the detection of the elongated oligonucleotides is carried out and visualized by means of matrix assisted laser desorption/ionization mass spectrometry (MALDI) or using electron spray mass spectrometry (ESI).

19. The method as recited in one of the preceding claims, wherein the polymerases are heat-resistant DNA-polymerases.

20. The method as recited in one of the preceding claims, wherein SNPs are also detected and visualized in addition to the DNA methylation.

21. The method as recited in one of the preceding claims, wherein the used nucleotides are terminating and/or chain-elongating nucleotides.

22. The method as recited in one of the preceding claims, wherein the nucleotides are selected from a group comprising either the nucleobases A, T and C or else the bases G and A and T.

23. The method as recited in claim 1, step a, wherein the genomic DNA is obtained from a DNA sample, sources of DNA comprising, e.g., cell lines, blood, sputum, stool, urine, cerebral-spinal fluid, tissue embedded in paraffin, histologic object slides, and all possible combinations thereof.

24. The method as recited in one of the preceding claims, **characterized in that** the methylation analyses of both DNA strands are carried out in one experiment.

## Revendications

1. Procédé de détection de 5-méthylcytosine ou de détection de 5-méthylcytosine et de SNP dans des échantillons d'ADN génomique, **caractérisé en ce qu'**on met en oeuvre les étapes suivantes:
(a) on fait réagir chimiquement avec un réactif un ADN génomique provenant d'un échantillon d'ADN, la 5-méthylcytosine et la cytosine réagissant différemment, et ces dernières présentant ainsi, après la réaction, un comportement différent d'appariement des bases dans le duplex d'ADN ;
(b) on amplifie l'ADN prétraité, par utilisation d'une polymérase et d'au moins une amorce oligonucléotidique ;
(c) on hybride l'ADN génomique amplifié, dans des conditions stringentes, à au moins un oligonucléotide ayant une séquence connue, ce à l'occasion de quoi, au moins entre une base dans le domaine 3' de l'oligonucléotide et la base devant être étudiée dans l'ADN, et selon l'état de méthylation de l'ADN initial, on aura un mésappariement, ou on n'aura aucun mésappariement ;
(d) on prolonge d'au moins un nucléotide à l'aide d'une polymérase les oligonucléotides qui dans le domaine 3' ne présentent aucun mésappariement avec la base devant être étudiée dans l'ADN, et d'au moins un nucléotide, au moins un nucléotide portant un marqueur détectable, tandis que les oligonucléotides qui dans le domaine 3' présentent un mésappariement avec la base devant être étudiée, ne sont pas prolongés ;
(e) on étudie les oligonucléotides prolongés, pour déterminer la présence du marqueur.

2. Procédé selon la revendication 1, **caractérisé en ce qu'**on lie sur des emplacements définis l'oligonucléotide ou les oligonucléotides selon la revendication 1 c, à une phase solide.

3. Procédé selon la revendication 1, **caractérisé en ce qu'**on lie à une phase solide les amplifiats sur des emplacements définis.

4. Procédé selon la revendication 2, **caractérisé en ce qu'**on dispose différentes séquences oligonucléotidiques sur une phase solide plane, sous forme d'un réseau rectangulaire ou hexagonal.

5. Procédé selon l'une des revendications 2 à 4, **caractérisé en ce que** la surface de la phase solide est constituée de silicium, de verre, de polystyrène, d'aluminium, d'acier, de fer, de cuivre, de nickel, d'argent ou d'or.

6. Procédé selon l'une des revendications 2 à 5, **caractérisé en ce que** les marqueurs rapportés aux oligonucléotides prolongés sont identifiables sur chaque position de la phase solide sur laquelle se trouve une séquence oligonucléotidique.

7. Procédé selon la revendication 1, **caractérisé en ce que**, lors de l'amplification, au moins une amorce qui n'est pas identique à l'oligonucléotide de la revendication 1 b se lie à une phase solide sur des emplacements définis.

8. Procédé selon la revendication 1, 3 ou 7, **caractérisé en ce qu'**on dispose des amplifiats différents sur la phase solide sous forme d'un réseau rectangulaire ou hexagonal.

9. Procédé selon l'une des revendications précédentes, **caractérisé en ce qu'**on met en oeuvre le traitement de l'ADN avant l'amplification avec une solution de bisulfite (= disulfite, hydrogénosulfite).

10. Procédé selon l'une des revendications précédentes, **caractérisé en ce que** l'amplification a lieu à l'aide de la réaction en chaîne par polymérase (PCR).

11. Procédé selon l'une des revendications précédentes, **caractérisé en ce que** les oligonucléotides utilisés dans la revendication 1 et spécifiés dans la revendication 7 ou bien ne contiennent que les bases T, A et C, ou bien cependant contiennent les bases T, A et G.

12. Procédé selon l'une des revendications précédentes, **caractérisé en ce que** les marqueurs des nucléotides sont des marqueurs par fluorescence.

13. Procédé selon l'une des revendications 1 à 11, **caractérisé en ce que** les marqueurs des nucléotides sont des radionucléides.

14. Procédé selon l'une des revendications 1 à 11, **caractérisé en ce que** les marqueurs des nucléotides sont des marqueurs de masse détachables, qui sont détectés dans un spectromètre de masse.

15. Procédé selon l'une des revendications 1 à 11, **caractérisé en ce qu'**on détecte globalement les oligonucléotides prolongés au spectromètre de masse, ces oligonucléotides étant ainsi marqués d'une manière non ambiguë par leur masse.

16. Procédé selon l'une des revendications 1 à 11, **caractérisé en ce qu'**on détecte dans chaque cas un fragment des oligonucléotides prolongés au spectromètre de masse.

17. Procédé selon l'une des revendications 1 à 16, **caractérisé en ce que**, pour une meilleure détectabilité au spectromètre de masse, les fragments produits présentent une charge nette individuelle positive ou négative.

18. Procédé selon l'une des revendications précédentes, **caractérisé en ce qu'**on met en oeuvre et on visualise la détection des oligonucléotides prolongés par une spectrométrie de masse par désorption-ionisation laser assistée par matrice (MALDI) ou par une spectrométrie de masse par électropulvérisation (ESI).

19. Procédé selon l'une des revendications précédentes, dans lequel les polymérases sont des ADN-polymérases thermostables.

20. Procédé selon l'une des revendications précédentes, dans lequel, outre la méthylation de l'ADN, on détecte et visualise aussi des SNP.

21. Procédé selon l'une des revendications précédentes, dans lequel les nucléotides utilisés sont des nucléotides terminaux et/ou des nucléotides prolongateurs de chaîne.

22. Procédé selon l'une des revendications précédentes, dans lequel les nucléotides sont choisis dans un groupe qui contient ou bien les nucléobases A, T et C, ou bien cependant les bases G et A et T.

23. Procédé selon la revendication 1, dans lequel l'ADN génomique a été obtenu à partir d'un échantillon d'ADN, les sources d'ADN comprenant par exemple les lignées cellulaires, le sang, le crachat, les selles, l'urine, le liquide céphalorachidien, un tissu enrobé de paraffine, un porte-objet histologique, et toutes les combinaisons de ceux-ci.

24. Procédé selon l'une des revendications précédentes, **caractérisé en ce qu'**on met en oeuvre simultanément dans une seule expérience les analyses de méthylation des deux brins d'ADN.
